# EUROPEAN PATENT APPLICATION

(11) **EP 4 831 289 A1**
(43) Date of publication of application: **28.10.2026**
(21) Application number: 24907528.4
(22) Date of filing: 20.12.2024
(51) Int. Cl.: C12N 15/13, C07K 16/00, C12N 5/00

(54) **METHOD FOR PRODUCING ANTIBODIES**

(30) Priority: 22.12.2023 WO PCT/JP2023/046062
(71) Applicant: CHUGAI SEIYAKU KABUSHIKI KAISHA, Tokyo 115-8543 (JP)
(72) Inventor: TOMIYAMA, Noribumi, Tokyo 115-8543 (JP)
(74) Representative: Vossius & Partner Patentanwälte Rechtsanwälte mbB
(86) International application number: PCT/JP2024/045095
(87) International publication number: WO 2025/135144

(57) **Abstract**

A method for generating a cell that produces a multispecific binding molecule, the method including a step of introducing a first vector and a second vector into a cell, wherein the first vector contains genes for all polypeptide chains constituting the multispecific binding molecule, the second vector contains a gene for a polypeptide chain with low expression, and the multispecific binding molecule may be a multispecific antibody.

## Description

### TECHNICAL FIELD

The present invention relates to a method for producing an antibody.

### BACKGROUND ART

When producing pharmaceutical recombinant antibodies using genetic recombination technology, mammalian cells are often used as host cells for recombinant antibody production. This is because, by using mammalian cells, it becomes possible to perform complex post-translational modifications and folding that cannot be performed with prokaryotic cells. Gene introduction into host cells is carried out by incorporating an antibody-producing gene into a plasmid to construct an expression vector, which is then introduced into mammalian cells.

Antibody drugs are produced using an immunoglobulin framework. There are five main classes of immunoglobulins: IgG, IgM, IgD, IgE, and IgA. Almost all of the antibody drugs approved so far contain sequences derived from IgG. The IgG-type antibody molecule contains four polypeptide chains (two H chains and two L chains). These polypeptide chains form a symmetrical Y-shaped structure with combination of non-covalent interactions and interchain covalent disulfide bonds. The IgG-type antibody molecule has an H chain known as γ chain. The L chain may be one of two types: either κ chain or λ chain.

Typically, to generate cells to produce recombinant antibodies, one copy of DNA encoding an H chain and one copy of DNA encoding an L chain of the antibodies are introduced into host cells. In contrast, WO 2009/051108 (PTL 1) describes an antibody production method where an expression vector containing one copy of DNA encoding an H chain and two copies of DNA encoding an L chain of a recombinant antibody is introduced into host cells.

WO 2006/060769 (PTL 2) describes a method for producing a heterodimeric or heteromultimeric protein using two types of expression vectors, each encoding a different polypeptide chain. When the protein is an immunoglobulin, the first vector encodes the heavy chain (H chain) and the second vector encodes the light chain (L chain). It describes that the optimal ratio of the heavy chain plasmid to the light chain plasmid for maximum antibody productivity has been investigated.

The basic structure of an IgG-type antibody molecule consists of two identical H chains and two identical L chains, forming a monospecific molecule with the same binding specificity on both arms (at the tips of the F(ab) regions). In contrast, in recent years, multispecific antibodies that allow a single antibody molecule to bind to multiple different targets have been developed (for example, WO 2006/109592 (PTL 3)). Examples of the multispecific antibodies include bispecific antibodies (BsAbs) that bind to two antigens and multispecific antibodies with a larger number of antigen-binding sites.

Bispecific antibodies have two different antigen specificities. Typically, IgG-type bispecific antibodies contain two different H chains and two different L chains. When these four types of polypeptide chains are expressed in cells, various different antibody species are produced due to the random association of the H chains and the L chains, resulting in the formation of nine types of impure antibodies in addition to the desired combination. The formation of impure antibodies not only reduces the yield of the desired bispecific antibody. Due to the similarity in properties among the ten types of antibodies that have been formed, it also causes a significant issue in industrial manufacturing that the separation and removal of impure antibodies is difficult using the conventional production methods of antibody drugs.

When making the H chains or L chains of a bispecific antibody common, three combinations of expression products (two types of monospecific antibodies and one type of bispecific antibody) are expected, allowing for increase in yield. There is a demand for technique that further increases the yield of the desired bispecific antibody and enables high-purity purification.

WO 2016/146594 (PTL 4) describes a method for purifying a bispecific antibody with a common H chain from a mixture of antibodies. This bispecific antibody is constructed from a single H chain and two different L chains. The host cells for the antibody production are transformed with an expression vector containing one DNA encoding a common H chain, one DNA encoding a first L chain, and one DNA encoding a second L chain (three-gene expression vector).

"Transient and Stable CHO Expression, Purification and Characterization of Novel Hetero-Dimeric Bispecific IgG Antibodies: Biotechnol. Prog., 2017, Vol. 33, No. 2, P469-477" (NPL 1) describes that, to produce a specific bispecific antibody using a single plasmid vector in CHO cells, a ratio of 60% for the common L chain and 20% for each H chain has been optimal.

"Optimizing assembly and production of native bispecific antibodies by codon de-optimization MABS 2017, VOL. 9, NO. 2, P231-P2397" (NPL 2) describes that in the production of bispecific antibodies with a common H chain, even though the expression levels of the poorly expressed arm have been increased through codon optimization, the yield of the desired bispecific antibody has not been improved.

"Tuning Relative Polypeptide Expression to Optimize Assembly, Yield and Downstream Processing of Bispecific Antibodies;Antibodies 2018, 7, 29" (NPL 3) describes that in the production of bispecific antibodies using a common H chain, the ratio of L chains has been changed in several ways.

### CITATION LIST

### PATENT LITERATURE

PTL 1: WO 2009/051108 (Japanese Patent No. 4976502)
PTL 2: WO 2006/060769 (Japanese Translation of PCT International Application Publication No. 2008-522589)
PTL 3: WO 2006/109592 (Japanese Patent No. 4917024)
PTL 4: WO 2016/146594 (Japanese Patent No. 6894843)

### NON PATENT LITERATURE

NPL 1: Transient and Stable CHO Expression, Purification and Characterization of Novel Hetero-Dimeric Bispecific IgG Antibodies: Biotechnol. Prog., 2017, Vol. 33, No. 2, P469-477
NPL 2: Optimizing assembly and production of native bispecific antibodies by codon de-optimization MABS 2017, VOL. 9, NO. 2, P231-P2397
NPL 3: Tuning Relative Polypeptide Expression to Optimize Assembly, Yield and Downstream Processing of Bispecific Antibodies;Antibodies 2018, 7, 29

### SUMMARY OF INVENTION

### TECHNICAL PROBLEM

An object of the present invention is to provide a method for producing a bispecific antibody with high production efficiency.

### SOLUTION TO PROBLEM

The present inventors have found that in the production of bispecific antibodies with a common L chain, the use of two types of expression vectors improves both the production yield and the selectivity of the desired heteromeric bispecific antibody, and have completed the present invention.

In other words, the present invention relates to constructing a cell line by transforming a host cell with two types of recombinant vectors to produce multispecific binding molecules such as bispecific antibodies, and using the obtained cell line to produce multispecific binding molecules composed of polypeptide chains of the desired combination. More specifically, the present invention can be expressed as follows.
(1) A method for generating a cell that produces a multispecific binding molecule, the method comprising a step of introducing a first vector and a second vector into a cell, wherein the first vector contains genes for all polypeptide chains constituting the multispecific binding molecule, and the second vector contains a gene for a polypeptide chain with low expression.
(2) The method according to (1), wherein the multispecific binding molecule is an antigen-binding molecule or antibody, the multispecific antigen-binding molecule or antibody may be a bispecific antibody having a common L chain or a common H chain, and the bispecific antibody may be an IgG-type antibody with an H chain or L chain made common.
(3) A method for generating a cell that produces a bispecific antibody with a common L chain, the method comprising a step of introducing a first vector and a second vector into a cell, wherein the first vector contains genes for all polypeptide chains constituting the bispecific antibody, and the second vector contains a gene for a polypeptide chain with low expression of the bispecific antibody.
(4) The method according to (3), wherein the polypeptide chain with low expression is one of two different H chains, and the second vector contains a nucleic acid sequence encoding the H chain and a nucleic acid sequence encoding the common L chain.
(5) The method according to (4), wherein the second vector contains a greater number of the nucleic acid sequence encoding the L chain polypeptide than the nucleic acid sequence encoding the H chain polypeptide.
(6) The method according to (5), wherein the first vector contains a nucleic acid sequence encoding a first H chain, a nucleic acid sequence encoding a second H chain, and a nucleic acid sequence encoding the common L chain in a ratio of 1 : 1 : 1 or 1 : 1 : 2, and the second vector contains a nucleic acid sequence encoding the H chain with lower expression and a nucleic acid sequence encoding the common L chain in a ratio of 1 : 2.
(7) The method according to (4), wherein the first vector contains one copy of a nucleic acid sequence encoding a first H chain, one copy of a nucleic acid sequence encoding a second H chain, and one or two copies of the nucleic acid sequence encoding the common L chain, and the second vector contains one copy of a nucleic acid sequence encoding an H chain with lower expression (either the first H chain or the second H chain) and two or more copies of the nucleic acid sequence encoding the common L chain.
(8) A cell that produces an antigen-binding molecule, the cell comprising a first vector and a second vector, wherein the first vector contains genes for all polypeptide chains constituting the antigen-binding molecule, the second vector contains a gene for a polypeptide chain with low expression, the antigen-binding molecule may be multispecific, or may be a bispecific antibody with an H chain or L chain made common.
(9) A cell that produces a bispecific antibody with a common L chain, the cell comprising a first vector and a second vector, wherein the first vector contains genes for all polypeptide chains constituting the bispecific antibody, and the second vector contains a gene for a polypeptide chain with low expression of the bispecific antibody.
(10) The cell according to (9), wherein the second vector contains a gene for an H chain with lower expression and further contains a gene for the common L chain, and the second vector may contain the gene for the common L chain at a higher copy number than the gene for the H chain.
(11) The cell according to (9), wherein the first vector contains one copy of a gene for a first H chain, one copy of a gene for a second H chain, and one or two copies of a gene for a common L chain, and the second vector contains one copy of the gene for an H chain with lower expression and two or more copies of the gene for a common L chain.
(12) A method for producing an antigen-binding molecule or bispecific antibody using any of the cell according to (1) to (11).
(13) The method for generating a cell, the cell, or the production method described above, wherein the cell produces an anti-FIX(a)/FX bispecific antibody such as emicizumab.
(14) The method for generating a cell, the cell, or the production method described above, wherein the cell is an animal cell, for example, a mammalian cell, preferably a CHO cell.
(15) A method for producing a medicament containing an antigen-binding molecule or a bispecific antibody produced by the method described above.

In the method for producing a cell described above, when the method is a method for producing a cell that produces a bispecific antibody having a common L chain, the "polypeptide with low expression" is a polypeptide constituting a monospecific antibody with lower expression level among two types of monospecific antibodies (i.e., impure antibodies) where the H chain is homogenous.

The method for producing a cell described above may include a selection step of selecting a clone with high expression of the desired gene, after introducing the first vector and the second vector into the cell.

The method for producing an antigen-binding molecule or bispecific antibody includes a culturing step of culturing a clone cell obtained by the method for producing a cell (i.e., a cell line that produces the desired antigen-binding molecule or bispecific antibody). Furthermore, the method for producing an antigen-binding molecule or bispecific antibody in the present invention may include a purification step of purifying the desired antigen-binding molecule or bispecific antibody from the culture obtained through the culturing step of the clone cell.

The present invention also encompasses a transformed cell obtained by the method for producing a cell described above. The transformed cell includes a first vector and a second vector described above. For example, a cell transformed to produce a bispecific antibody with a common L chain contains a first vector and a second vector, wherein the first vector is an expression vector containing exogenous DNA encoding all polypeptide chains constituting the bispecific antibody, and the second vector is an expression vector containing exogenous DNA encoding a polypeptide chain with low expression of the bispecific antibody, and the second vector may further contain exogenous DNA encoding another polypeptide chain.

### ADVANTAGEOUS EFFECTS OF INVENTION

According to the present invention, while increasing the expression level of bispecific antibody, the expression levels of two types of monospecific antibodies, which are impure antibodies, are also balanced, thus handling during purification becomes easier. Using the present invention, cells suitable for the production of bispecific antibodies can be generated, and the cells can be established as highly efficient cell lines for the production of antibody drugs.

### BRIEF DESCRIPTION OF DRAWINGS

Fig. 1 shows a schematic diagram of each chain arrangement in each of three types of plasmids.
Fig. 2 shows the heteromeric form production ratio of the cell pools into which each of the three different plasmids was introduced.

### DESCRIPTION OF EMBODIMENTS

### 1. Multispecific Binding Molecule

A multispecific binding molecule is a molecule that can bind to two or more different targets. The multispecific binding molecule contains different polypeptide chains, each containing a target binding site that recognizes different targets. Examples of the multispecific binding molecule include protein molecules containing two types of polypeptide chains, three types of polypeptide chains, four types of polypeptide chains, or a greater number of different polypeptide chains.

Examples of the multispecific binding molecule include an antigen-binding molecule. An antigen-binding molecule consists of a plurality of polypeptide chains. A multispecific binding molecule can be a multispecific antigen-binding molecule that has at least a first antigen-binding site that recognizes a first antigen and a second antigen-binding site that recognizes a second antigen. Preferable examples of the multispecific antigen-binding molecule include a multispecific antibody that can specifically bind to at least two different antigens. A multispecific antibody may contain a first antigen-binding site, a second antigen-binding site, and optionally, a third antigen-binding site.

In the present invention, the term "different antigen" does not necessarily mean that the antigen itself needs to be different, and cases where the epitopes are different are also included as "different antigens". Thus, for example, different epitopes in a single molecule are also included as "different antigens".

As a preferred multispecific antibody in the present invention, a bispecific antibody (also sometimes referred to as a dual-specific antibody) capable of specifically binding to two different antigens can be mentioned.

The bispecific antibody can be an IgG-type antibody molecule composed of, for example, two different heavy chains (H chains) and two different light chains (L chains), namely, four types of polypeptide chains. Alternatively, the bispecific antibody can be an IgG-type antibody with an H chain or L chain made common.

In one embodiment of the present invention, the light chain (L chain) of the bispecific antibody may be different, but it is preferable to have a light chain that is in common (referred to as "common L chain"). The "common L chain" associates with two or more different H chains and can exhibit binding ability to each respective antigen. Here, the "different H chains" preferably refer to the H chains of antibody against different antigens, but are not limited to this, and mean H chains that have different amino acid sequences from each other. The common L chain can be obtained, for example, by the method described in WO 2006/109592 (PTL 3).

A bispecific antibody is an immunoglobulin molecule that has specificity for two different antigens. The origins of the polypeptide chains constituting the antibody are not particularly limited and the polypeptide chains can be derived from humans, mice, rats, or the like. It is acceptable for the H chain and L chain originating from different animal species to coexist in the same antibody. Each polypeptide chain may be modified to a chimeric state using recombinant DNA technology, and may be a synthesized immunoglobulin chain. In one embodiment, the bispecific antibody of the present invention is an antibody modified using recombinant DNA technology. In one embodiment, the bispecific antibody of the present invention is a native antibody. The bispecific antibody of the present invention is preferably a full-length IgG-type antibody.

The "full-length antibody", "complete antibody", and "entire antibody" are used interchangeably herein to refer to antibodies that have a structure substantially similar to that of a native antibody. The full-length antibody has a heavy chain that contains an Fc region.

The native antibody refers to an immunoglobulin molecule that occurs naturally with various structures. As a general example, a native IgG-type antibody is a heterotetrameric glycoprotein of about 150,000 daltons, composed of two identical light chains (L chains) and two identical heavy chains (H chains) linked by disulfide bonds. From the N-terminus to the C-terminus, each heavy chain has a variable region (VH) also known as a variable heavy chain domain or heavy chain variable domain, followed by three constant domains (CH1, CH2, and CH3). Similarly, from the N-terminus to the C-terminus, each light chain has a variable region (VL) also known as the variable light chain domain or light chain variable domain, followed by a constant light chain (CL) domain. The light chain of an antibody can be classified into one of two types, kappa (κ) and lambda (λ), based on the amino acid sequence of its constant domain. A native IgG-type antibody, composed of two identical light chains (L chains) and two identical heavy chains (H chains), is a molecule of single specificity and is not a "multispecific binding molecule" in the context of the present invention.

### 2. Construction of cell line producing multispecific binding molecule

The present invention provides a method for generating a cell that produces a multispecific binding molecule, using two vectors. The method includes a step of introducing a first vector and a second vector into a cell, wherein the first vector contains genes for all polypeptide chains constituting the multispecific binding molecule, and the second vector contains a gene for a polypeptide chain with low expression.

In the present invention, a "polypeptide chain with low expression" refers to a polypeptide chain that has a lower expression level compared to other polypeptide chains when a plurality of genes for polypeptide chains constituting a multispecific binding molecule are introduced into the same cell. For instance, in a bispecific antibody with a common L chain, it is possible to identify a polypeptide chain with low expression by comparing the amount of homomeric antibodies formed by the association of the same polypeptide chains (i.e., the same H chains and common L chain) other than the desired bispecific antibody. In the case of a bispecific antibody with a common L chain, the second vector is a vector containing the genes encoding the H chain with low expression and the common L chain.

The term "vector" refers to a nucleic acid molecule that can increase another nucleic acid to which it is linked. The term generally includes a vector as a self-replicating nucleic acid structure and a vector that is incorporated into the genome of a host cell into which it has been introduced. A certain vector can lead to an expression of a gene nucleic acid to which the vector is operatively linked. Such a vector is also referred to as an "expression vector".

In the present invention, a vector containing a gene of a polypeptide chain refers to an expression vector that contains a nucleic acid sequence encoding an amino acid sequence of the polypeptide. By bringing such a vector into contact with any suitable host cell, the foreign gene encoding the desired polypeptide is introduced, and the cell becomes a transformed cell capable of producing the polypeptide.

Any suitable method can be used to bring cells into contact with vectors so that the cells express the polypeptides encoded by the genes contained in each vector. The method of bringing cells into contact so that the cells are modified to express specific polypeptides are well-known in the art. Examples of the suitable method of bringing cells into contact include infection with a viral vector, transfection using calcium chloride method, lipofection reagent, cationic polymer, DEAE, or calcium phosphate, and electroporation. By bringing the host cell into contact with the vector, the vector is introduced into the host cell.

In the step of introducing the two vectors into the cells by bringing the cells into contact with these vectors, the order of contact between the cells and each vector is not limited. To achieve the contact between the cells and the two vectors, the cells can be brought into contact with the first vector and the second vector sequentially (for example, bringing the cells into contact with the second vector after the first vector, or bringing the cells into contact with the second vector before the first vector), or the cells can be brought into contact with both the first vector and the second vector simultaneously.

In the present invention, the origin of the cells brought into contact with the vectors for the purpose of producing a protein that is a multispecific binding molecule, that is, the cells into which genes of the polypeptide chains constituting the multispecific binding molecule are introduced and which become the host cell line that produces the multispecific binding molecule, is not particularly limited. The cells may be adherent cells or suspended cells (i.e., cells that grow in suspension). Both eukaryotic microorganisms such as bacteria, filamentous fungi or yeast, and cells derived from multicellular organisms (invertebrates and vertebrates) can be used as host cells. Examples of invertebrate cells include plant and insect cells.

In the present invention, vertebrate cells can be suitably used as hosts. For example, a mammalian cell line adapted to proliferate in a suspended state would be useful. Mammalian cells suitable for generating cell lines for protein production are known in the art, and examples thereof include SV40-transformed simian kidney CV1 cells (COS-7); human fetal kidney cells (293 cells such as those described in Graham et al., J. Gen Virol. 36:59 (1977)); baby hamster kidney cells (BHK); mouse Sertoli cells (TM4 cells such as those described in Mather, Biol. Reprod. 23:243-251 (1980)); simian kidney cells (CV1); African green monkey kidney cells (VERO-76); human cervical cancer cells (HELA); canine kidney cells (MDCK); Buffalo rat liver cells (BRL 3A); human lung cells (W138); human liver cells (Hep G2); mouse mammary tumor (MMT 060562); TRI cells (for example, those described in Mather et al., Annals N.Y. Acad. Sci. 383:44-68 (1982)); MRC5 cells; and FS4 cells. Other examples of useful mammalian host cell lines include Chinese hamster ovary (CHO) cells including DHFR- CHO cells (Urlaub et al., Proc. Natl. Acad. Sci. USA 77:4216 (1980)); and myeloma cell lines such as Y0, NS0, and Sp2/0. For a general review of specific mammalian host cell lines suitable for antibody production, see, for example, Yazaki and Wu, Methods in Molecular Biology, Vol. 248 (B.K.C. Lo, ed., Humana Press, Totowa, NJ), pp. 255-268 (2003).

Examples of suitable host cells for the production of antibody drugs with a full-length IgG framework include the aforementioned human cells or non-human mammalian cells. On the other hand, as host cells used for the purpose of amplifying or replicating (cloning) recombinant expression vectors, prokaryotic cells (for example, E. coli) are preferably used.

The terms "host cell", "host cell line", and "host cell culture" are used interchangeably and refer to cells into which foreign nucleic acids have been introduced (including the progeny of such cells). The host cells include "transformants" and "transformed cells", which encompasses the primary transformed cells as well as their progeny derived from those cells, irrespective of the passage number. The progeny does not necessarily have to be completely identical to the parent cell in terms of nucleic acid content and may include mutations. The host cells also include mutant progeny that have the same function or biological activity as those used when the original transformed cells had been screened or selected.

A recombinant antibody is generally obtained by cloning DNA that encodes the antibody with antibody-producing cells such as hybridomas or antibody-producing sensitized lymphocytes, incorporating the DNA into an appropriate vector, and introducing the vector into host cells for production. An IgG-type bispecific antibody can be produced by introducing and co-expressing genes for the L chains and H chains that constitute the desired two IgG, a total of four genes, into cells. When a bispecific antibody has an L chain in common, a bispecific IgG can be expressed by introducing genes for the common L chain and two H chains, a total of three genes, into cells to express IgG.

Assuming that the expression levels of the common L chain and two H chains are equal, when using a vector containing DNA encoding the first H chain, DNA encoding the second H chain, and DNA encoding the common L chain in a ratio of 1 : 1 : 2, the three types of polypeptide chains expressed in this ratio are expected to associate, resulting in the desired bispecific IgG (i.e., IgG with heterologous H chains) obtained at a ratio of 50% and respective monospecific IgG with identical H chains obtained at a ratio of 25% each. However, there are cases where not all polypeptide chains constituting a bispecific antibody are expressed at the same level. For example, when the expression level of one of the two types of H chains is lower than that of the other H chain, the production levels of the two types of homomeric forms may differ.

In one embodiment, the present invention provides a novel method for producing cells that produce bispecific antibodies with a common L chain. This method includes a step of introducing a first vector and a second vector into the cells. The first vector is a vector containing genes of all polypeptide chains that constitute the bispecific antibody. Accordingly, the first vector contains all three genes that respectively encode the first H chain, the second H chain, and the common L chain of the bispecific antibody. For example, the first vector contains three genes encoding the first H chain, the second H chain, and the common L chain of the bispecific antibody at copy number ratios of 1 : 1 : 1 or 1 : 1 : 2. For example, the first vector contains one copy of the gene for the first H chain, one copy of the gene for the second H chain, and one or two copies of the gene for the common L chain. The second vector is a vector containing a gene of a polypeptide with low expression of the bispecific antibody. Among the polypeptides constituting the bispecific antibody with a common L chain, the polypeptide with low expression may be either the first H chain or the second H chain. Thus, the second vector contains a gene encoding either the first H chain or the second H chain.

If necessary, the second vector may also contain a gene that encodes a common L chain. The second vector may contain a greater number of genes encoding the common L chain than the genes encoding the H chain. For example, the second vector can contain the gene encoding either the first H chain or the second H chain and the gene encoding the common L chain at a copy number ratio of 1 : 1 or 1 : 2. For example, the second vector can contain one copy of a gene for the H chain with lower expression and one copy or two copies or two or more copies of a gene for the common L chain.

In one embodiment of the present invention, the cells that produce a bispecific antibody with a common L chain are transformed with a first vector and a second vector. The first vector is a vector that encodes all polypeptide chains constituting the bispecific antibody, and the second vector can contain a greater number of nucleic acid sequences encoding the polypeptide of the L chain than the nucleic acid sequences encoding the polypeptide of the H chain. For example, the second vector can contain one copy of a gene encoding either H chain and two copies or two or more copies of a gene encoding the common L chain.

In the present invention, cells producing the bispecific antibody with a common L chain are preferably cells suitable for the production of recombinant proteins used as medicaments. Mammalian cells suitable for the construction of cell lines for recombinant protein production are as previously described above.

In the present invention, the two vectors (i.e., the first vector and the second vector) used to generate cells that produce multispecific binding molecules or bispecific antibodies can be, for example, plasmid vectors or viral vectors. In the present invention, the vector, for example, may contain control sequences such as start and stop codons for transcription and translation (according to the host), appropriate promoters, and marker genes that enable selection of a host into which the foreign gene has been introduced, if necessary. Furthermore, the vector, for example, may contain an antibody signal sequence that promotes the secretion of antibodies into the extracellular environment. In the present invention, for example, the vector can be designed for the purpose of transient expression or stable expression. A person skilled in the art can appropriately construct a recombinant expression vector according to the purpose using known standard techniques.

In the present invention, the first vector and the second vector can independently be any type of vector. The first vector and the second vector may have identical control sequences, suitable promoters, marker genes, or the like, and only the recombinant-polypeptide coding sequences contained therein may differ.

The vectors that can be used in the present invention will be described in further detail. For example, when using E. coli as a host, it is preferable that the vector has an "ori" for amplification in E. coli and a selectable marker gene for the selection of transformed E. coli, which is preferably a drug resistance gene that allows for identification through certain drugs such as ampicillin, tetracycline, kanamycin, or chloramphenicol, to enable large-scale amplification and preparation of the vector in E. coli (e.g., JM109, DH5α, HB101, XL1Blue). Examples of the vectors include M13 series vectors, pUC series vectors, pBR322, pBluescript, and pCR-Script. For the purpose of subcloning and excising cDNA, examples include, in addition to the aforementioned vectors, pGEM-T, pDIRECT, and pT7.

When using vectors for the purpose of producing multispecific binding molecules or bispecific antibodies, expression vectors are particularly useful. For the expression vectors, for example, to achieve expression in E. coli, in addition to the aforementioned characteristics that allow the vectors to be amplified in E. coli, it is preferable that the vectors have a promoter that allows efficient expression in E. coli when the host is E. coli such as JM109, DH5α, HB101, or XL1-Blue. Examples of the promoter include lacZ promoter (Ward et al., Nature (1989) 341, 544-546; FASEB J. (1992) 6, 2422-2427), araB promoter (Better et al., Science (1988) 240, 1041-1043), and T7 promoter. Examples of such vectors include, in addition to the aforementioned vectors, pGEX-5X-1 (manufactured by Pharmacia Corporation), "QIAexpress system" (manufactured by QIAGEN N.V.), pEGFP, and pET (in this case, a host expressing T7 RNA polymerase such as BL21 is preferred).

The vector may contain a signal sequence for polypeptide secretion. For the signal sequence for polypeptide secretion, when producing in the periplasm of E. coli, the pelB signal sequence (Lei, S. P. et al J. Bacteriol. (1987) 169, 4379) may be used.

In addition to the vectors when using Escherichia coli as a host, examples of the vectors that can be used in the method of the present invention include expression vectors derived from mammals (e.g., pcDNA3 (manufactured by Invitrogen) or pEGF-BOS (Nucleic Acids. Res. 1990, 18(17), p5322), pEF, pCDM8, or INPEP4 (manufactured by Biogen-IDEC)), expression vectors derived from insect cells (e.g., "Bac-to-BAC baculovirus expression system" (manufactured by GIBCO BRL), pBacPAK8), expression vectors derived from plants (e.g., pMH1, pMH2), expression vectors derived from animal viruses (e.g., pHSV, pMV, pAdexLcw), expression vectors derived from retroviruses (e.g., pZIpneo), expression vectors derived from yeast (e.g., "Pichia Expression Kit" (manufactured by Invitrogen), pNV11, SP-Q01), and expression vectors derived from Bacillus subtilis (e.g., pPL608, pKTH50).

To achieve expression in animal cells such as CHO cells, COS cells, and NIH3T3 cells, it is preferable that the vectors have a promoter necessary for expression within the cells, such as SV40 promoter (Mulligan et al., Nature (1979) 277, 108), MMLV-LTR promoter, EF1α promoter (Mizushima et al., Nucleic Acids Res. (1990) 18, 5322), CMV promoter (Niwa et al., Gene. (1991) 108, 193), mouse β-globin promoter (mBGP), and CAG promoter (Niwa et al., Gene. (1991) 108, 193, Miyazaki et al., Gene (1989) 79, 269), and more preferable to have a gene for selecting transformants in cells, such as a drug resistance gene that can be identified using drugs (e.g., neomycin and G418). Examples of vectors with such characteristics include pMAM, pDR2, pBK-RSV, pBK-CMV, pOPRSV, and pOP13. It is known that mRNA with polyA is stabilized within cells, and it is preferable to have a polyA signal necessary for adding polyA to the gene, such as mouse β-globin polyA signal, bovine growth hormone polyA signal (rBG-pA), or SV40 polyA signal.

In one embodiment of the present invention, for expression in CHO cells, a neomycin resistance gene, CAG promoter, rBG-pA, and the like can be suitably used. The recombinant proteins produced using CHO cells have been confirmed to be safe for use as medicaments and this method has become common. One preferred embodiment for producing a bispecific antibody as an active ingredient in a medicament using the present invention is a recombinant protein expression system using CHO cells.

The means to deliver genes into cells are well known in the art and can be appropriately selected according to the cells used as hosts. Commercially available gene introduction systems may also be used. Examples of the method for introducing vectors into mammalian cells include electroporation, methods of using transfection reagents such as lipofection, and methods of using viral vectors. Examples of the method for inserting foreign genes into the host genome of mammalian cells include random integration, Targeted Integration (site-specific gene insertion using recombinase that is a sequence-specific recombination enzyme), transposon vectors, and methods using site-specific nucleases. The site-specific gene introduction method to specific positions of the host genome is expected to be an efficient method for obtaining cells with superior production and passage stability of the desired protein.

The cells of the present invention may express antigen-binding molecules (antibodies) in a transient expression system or in a stable expression system, but those express them in a stable expression system are preferred.

The transient expression system is a method of introducing and expressing a circular plasmid inside cells using techniques such as the calcium phosphate method, electroporation method, and lipofection method. Circular plasmids have a low efficiency of being inserted into chromosomes, and the desired gene often exists outside the chromosomes. For this reason, it is difficult to maintain the expression of the desired gene from the circular plasmid for a long period.

The stable expression system is a method of introducing and expressing a linear plasmid created by restriction enzyme digestion treatments or the like into cells using techniques such as the calcium phosphate method, electroporation method, and lipofection method. Linear plasmids have a higher efficiency of integration into chromosomes compared to circular plasmids, and the efficiency of maintaining the desired gene on the chromosome increases. This allows for the long-term maintenance of the expression of the desired gene. Furthermore, the introduction of drug resistance genes into plasmids allows for drug selection, enabling the efficient selection of cells in which the desired gene is maintained on the chromosome. Examples of animal cells used in the stable expression system include CHO cells, NS0 cells, and SP2/0 cells, with CHO cells being preferred.

Furthermore, to achieve stable gene expression and amplification of gene copy number within cells, a method including introduction of a vector with a DHFR gene that complements the deficiency in the nucleic acid synthesis pathway in CHO cells (for example, pCHOI) into CHO cells lacking such a pathway, and then amplifying it using methotrexate (MTX) can be employed. Additionally, to achieve transient gene expression, a method including the use of COS cells that carry a gene expressing SV40 T antigen on the chromosome and conducting transformation with a vector (such as pcD) containing SV40 replication origin can be employed. As a replication origin, those derived from polyomavirus, adenovirus, bovine papillomavirus (BPV) or the like can also be used. Furthermore, for the purpose of gene copy number amplification in host cell lines, the expression vector can contain selection markers such as the aminoglycoside transferase (APH) gene, thymidine kinase (TK) gene, E. coli xanthine-guanine phosphoribosyl transferase (Ecogpt) gene, and dihydrofolate reductase (dhfr) gene.

### 3. Production of antigen-binding molecules (culturing step)

The present invention further provides a method for producing a desired antigen-binding molecule by using cells that produce the aforementioned multispecific binding molecules or cells that produce bispecific antibodies with a common L chain.

The production of antigen-binding molecules can be performed by culturing the aforementioned cells under conditions suitable for production of antibodies. The method of culturing cells is known in the art. The conditions under which cells are cultured vary depending on the type of cells. The conditions include various factors such as the temperature of the environment, the culture vessel containing the cells, the composition of various gases (e.g., CO₂) that make up the cell culture atmosphere or environment, the culture medium, the cell density, and the schedule for replacing the culture medium with a fresh one. These parameters are known in the art or can be empirically determined. For example, the cells can be cultured in a medium using any method such that the cells express (and, in some cases, secrete) the polypeptide encoded by the vector that has been brought into contact with the cells.

For cell culture, a medium used in normal cell (preferably animal cell) culture can be used. For example, as a culture medium for animal cells, DMEM, MEM, RPMI1640, and IMDM can be used. It is also possible to use commercially available animal cell culture media such as D-MEM (Dulbecco's Modified Eagle Medium), D-MEM/F-12 1:1 Mixture (Dulbecco's Modified Eagle Medium : Nutrient Mixture F-12), RPMI1640, CHO-S-SFM II (Invitrogen), CHO-SF (Sigma-Aldrich Co. LLC), EX-CELL 301 (JRH Biosciences), CD-CHO (Invitrogen), IS CHO-V (Irvine Scientific), and PF-ACF-CHO (Sigma-Aldrich Co. LLC). These media can be supplemented with serum supplements such as fetal calf serum (FCS), or they can be used for serum-free culture.

The culture medium typically contains amino acids, vitamins, lipid factors, energy sources, osmotic regulators, iron sources, pH buffers, and, in some cases, for example, trace metal elements, surfactants, growth aid factors, and nucleosides. The appropriate concentration of these components is typically in the following ranges: amino acids 0.05-1500 mg/L, vitamins 0.001-10 mg/L, lipid factors 0-200 mg/L, energy sources 1-20 g/L, osmotic regulators 0.1-10000 mg/L, iron sources 0.1-500 mg/L, pH buffers 1-10000 mg/L, trace metal elements 0.00001-200 mg/L, surfactants 0-5000 mg/L, growth aid factors 0.05-10000 µg/L, and nucleosides 0.001-50 mg/L. However, these concentrations are not limited to these ranges and can be appropriately determined based on the type of cultured cells, the type of desired antigen-binding molecules, and the like.

More specifically, examples include a medium containing amino acids such as L-alanine, L-arginine, L-asparagine, L-aspartic acid, L-cysteine, L-cystine, L-glutamine, L-glutamic acid, glycine, L-histidine, L-isoleucine, L-leucine, L-lysine, L-methionine, L-ornithine, L-phenylalanine, L-proline, L-serine, L-threonine, L-tryptophan, L-tyrosine, and L-valine, preferably L-alanine, L-arginine, L-asparagine, L-aspartic acid, L-cystine, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, and L-valine; vitamins such as i-inositol, biotin, folic acid, lipoic acid, nicotinamide, niacin, p-aminobenzoic acid, calcium pantothenate, pyridoxal hydrochloride, pyridoxine hydrochloride, riboflavin, thiamine hydrochloride, vitamin B12, and ascorbic acid, preferably biotin, folic acid, lipoic acid, nicotinamide, calcium pantothenate, pyridoxal hydrochloride, riboflavin, thiamine hydrochloride, vitamin B12, and ascorbic acid; lipid factors such as choline chloride, choline tartrate, linoleic acid, oleic acid, and cholesterol, preferably choline chloride; energy sources such as glucose, galactose, mannose, and fructose, preferably glucose; osmotic regulators such as sodium chloride, potassium chloride, and potassium nitrate, preferably sodium chloride; iron sources such as ferric EDTA, ferric citrate, ferrous chloride, ferric chloride, ferrous sulfate, ferric sulfate and ferric nitrate, preferably ferric chloride, ferric EDTA, and ferric citrate; and pH buffers such as sodium bicarbonate, calcium chloride, sodium dihydrogen phosphate, HEPES, and MOPS, preferably sodium bicarbonate.

In addition to the above components, the medium may contain trace metal elements such as copper sulfate, manganese sulfate, zinc sulfate, magnesium sulfate, nickel chloride, tin chloride, magnesium chloride, and sodium metasilicate, preferably copper sulfate, zinc sulfate, and magnesium sulfate; surfactants such as Tween 80 and Pluronic(R) F68.; growth aid factors such as recombinant insulin, recombinant IGF-1, recombinant EGF, recombinant FGF, recombinant PDGF, recombinant TGF-α, ethanolamine hydrochloride, sodium selenite, retinoic acid, and putrescine hydrochloride, preferably sodium selenite, ethanolamine hydrochloride, recombinant IGF-1, and putrescine hydrochloride; and nucleosides such as deoxyadenosine, deoxycytidine, deoxyguanosine, adenosine, cytidine, guanosine, and uridine. In an appropriate example of the above-mentioned medium, the medium may contain antibiotics such as streptomycin, penicillin G potassium, and gentamicin, and pH indicators such as phenol red.

The pH of the medium varies depending on the cells being cultured, but it is preferably around 6 to 8. Generally, a pH of 6.8 to 7.6 is appropriate, and in many cases, a pH of 7.0 to 7.4 is optimal. Culture is usually performed at about 30 to 40°C for about 15 to 200 hours, with additionally performing media exchange, aeration, and stirring, as needed.

When the cells are CHO cells, culturing of CHO cells can be performed using methods known to those skilled in the art. For example, it is typically possible to culture under an atmosphere of a gaseous phase CO₂ concentration of 0-40%, preferably 2-10%, at 30-39°C, preferably around 37°C. The culture period of suitable cells for producing the desired antigen-binding molecules (antibodies or fragments thereof) is usually 1 day to 3 months, preferably 1 day to 2 months, and more preferably 1 day to 1 month.

Examples of various culture apparatuses for animal cell culture can include fermenter-type tank culture apparatus, air-lift type culture apparatus, culture flask-type culture apparatus, spinner flask-type culture apparatus, microcarrier-type culture apparatus, fluidized bed-type culture apparatus, hollow fiber-type culture apparatus, roller bottle-type culture apparatus, and packed bed-type culture apparatus.

Culture can be performed using any method such as batch culture, fed-batch culture, or continuous culture. However, fed-batch culture or continuous culture is preferred, with fed-batch culture being more preferable.

When producing a bispecific antibody as the active ingredient of a medicament, it is desirable for the desired bispecific antibody to be secreted into the medium through cell culture. It is then desirable that the association of the L chain and H chain proceeds naturally in the culture of antibody-producing cells.

The H-chain polypeptide and L-chain polypeptide that compose the antibody molecule assemble with the support of BiP (Immunoglobulin heavy chain binding protein), and then complete the full antibody structure by folding. This assembly step is dependent on L-chain polypeptides (Molecular Biology of the Cell, 1999, 10, 2209). It is thus considered that by increasing the ratio of L-chain gene numbers and the ratio of L-chain polypeptides, the assembly of H-chain polypeptides and L-chain polypeptides is promoted, thereby increasing the production yield.

### 4. Production of antigen-binding molecules (purification step)

The culture supernatant of animal cells contains impurities such as components secreted from the production cells other than the desired antigen-binding molecules (in the narrow sense, antibodies). Thus, purification is performed to remove impurities and extract the desired protein. The high-purity protein obtained through these culturing and purification steps is referred to as "active pharmaceutical ingredient". It then undergoes formulation steps (addition of additives, aseptic filtration, or the like) and filling and packaging steps to become a medicament.

Purification can be performed at any point after culturing the cells. The method for purifying proteins from culture medium or culture supernatant is known in the art. Examples of the appropriate purification method include chromatography and electrophoresis.

The separation and purification of antigen-binding molecules such as antibodies may be performed using the same separation and purification methods that are used conventionally for polypeptides. For example, antibodies can be separated and purified by appropriately selecting and combining chromatography columns such as affinity chromatography, filters, ultrafiltration, salting out, dialysis, SDS polyacrylamide gel electrophoresis, isoelectric focusing, and the like (Antibodies: A Laboratory Manual. Ed Harlow and David Lane, Cold Spring Harbor Laboratory, 1988), but separation and purification methods are not limited to these. The concentration measurement of the antibody obtained above can be performed by measuring absorbance or using enzyme-linked immunosorbent assay (ELISA), or the like.

Columns used for affinity chromatography include, for example, protein A columns and protein G columns. Examples of columns using protein A columns include Hyper D, POROS, and Sepharose F. F. (Pharmacia Corporation).

Examples of the chromatography other than affinity chromatography include ion exchange chromatography, hydrophobic chromatography, gel filtration, reversed-phase chromatography, and adsorption chromatography (Strategies for Protein Purification and Characterization: A Laboratory Course Manual. Ed Daniel R. Marshak et al., Cold Spring Harbor Laboratory Press, 1996). These chromatographies can be performed using liquid chromatography such as HPLC and FPLC. By performing purification using these chromatographies, the active pharmaceutical ingredient of the antibody drug is obtained.

Furthermore, by applying an appropriate polypeptide-modifying enzyme either before or after purification of the polypeptide, it is possible to add modifications or remove parts of peptides as desired. Examples of the polypeptide modifying enzyme include trypsin, chymotrypsin, lysyl endopeptidase, protein kinase, and glycosidase.

By using the cells of the present invention, the ratio of the amount of antibody-type molecules produced is constant, making it easy to separate and remove impure antibodies, thus obtaining antigen-binding molecules (antibodies) with the desired multispecificity at high purity. A typical example of the desired antigen-binding molecule is an IgG-type antibody molecule with a common L chain, including three types of polypeptide chains (i.e., a common L chain and two different H chains). The antibodies with combination including two identical H chains are impure antibodies.

### 5. Specific bispecific antibody

Emicizumab (also known as Ace910 or under the product name HEMLIBRA(R)) is a recombinant humanized bispecific monoclonal antibody against activated blood coagulation factor IX (F IX(a)) and blood coagulation factor X (F X). By binding one arm of the antibody to F IX(a) and the other arm to F X, and finely cross-linking F IX(a) and F X, it is used as a pharmaceutical to substitute the function of activated blood coagulation factor VIII (FVIIIa), which is deficient or dysfunctional in hemophilia A (Sampei, et al. PLoS ONE 2013; 8(2): e57479;Kitazawa, et al. Nature Medicine 2012; 18(10): 1570).

Emicizumab is a bispecific antibody against FIX(a)/FX with a common L chain. Emicizumab contains an H-chain polypeptide with an antigen-binding site recognizing F IX(a), an H-chain polypeptide with an antigen-binding site recognizing F X, and an L-chain polypeptide in common. The sequences of these polypeptides are known (in sequences of WO 2012/067176, SEQ ID NO. 20 represents the heavy chain on the FIX side, SEQ ID NO. 25 represents the heavy chain on the FX side, and SEQ ID NO. 32 represents the common L chain).

The present inventors have previously succeeded in increasing the production ratio of heteromeric forms (anti-FIX(a)/FX bispecific antibodies) by controlling the charge of each chain of antibodies and adopting a common L chain. However, in the plasmid encoding the H chain recognizing F IX, the H chain recognizing F X and the common L chain, F IX homomeric form (anti-F IX antibodies) are produced in higher amounts and F X homomeric form (anti-F X antibodies) are produced in lower amounts.

Accordingly, various investigations have been conducted to obtain an improved cell line in terms of heteromeric form production during creating the Working Cell Bank (WCB) at the manufacturing stage. As a result, by constructing a cell line using a plasmid introduced with FIX H chain : FX H chain : common L chain in a 1 : 1 : 2 ratio + a secondary plasmid introduced with H chain of lower homomeric form production rate (anti-F X antibody) : common L chain in a 1 : 2 ratio, a method of constructing a cell line that achieves both high production and selectivity for heteromeric forms has been found.

Accordingly, in a preferred embodiment of the present invention, a method for producing a cell that produces emicizumab, including a step of introducing a first vector and a second vector into the cell, is provided. In this method, the first vector contains all polypeptide chains constituting emicizumab, i.e., genes for FIX H chain, FX H chain, and common L chain at 1 : 1 : 1 or 1 : 1 : 2 in the copy number, and the second vector may contain a gene for FX H chain and, optionally, a gene for the common L chain. For example, the second vector can contain the FX H chain gene and the common L chain gene at 1 : 1 or 1 : 2 in the copy number. In certain embodiments, the first vector contains one copy of a gene for the FIX H chain, one copy of a gene for the FX H chain, and two copies of a gene for the common L chain, while the second vector contains one copy of a gene for the FX H chain and two or more copies of a gene for the common L chain.

### 6. Production of medicament

When the antigen-binding molecule or bispecific antibody produced by the method of the present invention possesses biological activity suitable for pharmaceutical use, a medicament can be produced by mixing and formulating the antigen-binding molecule or bispecific antibody with a pharmaceutically acceptable carrier or additive.

Examples of the pharmaceutically acceptable carrier or additive include water, pharmaceutically acceptable organic solvents, collagen, polyvinyl alcohol, polyvinylpyrrolidone, carboxyvinyl polymers, sodium carboxymethylcellulose, sodium polyacrylate, sodium alginate, water-soluble dextran, sodium carboxymethyl starch, pectin, methylcellulose, ethylcellulose, xanthan gum, gum arabic, casein, agar, polyethylene glycol, diglycerin, glycerin, propylene glycol, petroleum jelly, paraffin, stearyl alcohol, stearic acid, human serum albumin (HSA), mannitol, sorbitol, lactose, and surfactants acceptable as pharmaceutical additives.

In practice, additives are selected individually or in appropriate combinations from the above according to the dosage form of the medicament, but they are not limited to these. For instance, when used as an injectable preparation, the purified polypeptide can be dissolved in a solvent such as saline, buffer solution, or glucose solution, and to the resulting solution, an anti-adsorption agent such as Tween 80, Tween 20, gelatin, or human serum albumin can be added. Alternatively, it may be lyophilized for the purpose of forming a dosage form that requires dissolution and reconstitution before use. As excipients used for lyophilization, sugar alcohols or sugars such as mannitol and glucose can be used.

Hereinafter, the present invention will be specifically described by Examples. It should be noted that these Examples are for illustrative description of the present invention and do not limit the scope of the present invention.

### EXAMPLES

### Example 1: Gene introduction using one type of plasmid each

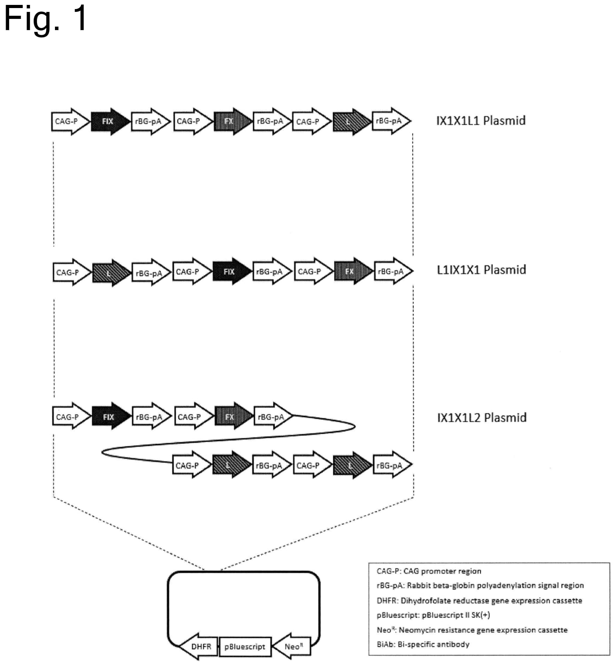

An FIX H chain expression unit, an FX H chain expression unit, and a common L chain expression unit were created by linking a CAG promoter upstream of each of the structural genes for Emicizumab FIX H chain, FX H chain, and common L chain, and further linking rBG-pA downstream thereof. The FIX H chain expression unit, the FX H chain expression unit, and the common L chain expression unit were inserted into pBluescriptII containing a neomycin resistance gene to construct an IX1X1L1 plasmid consisting of 1 copy for the FIX H chain, 1 copy for the FX H chain, and 1 copy for the common L chain, an L1IX1X1 plasmid where the position of the L chain was swapped, and an IX1X1L2 plasmid consisting of 1 copy for the FIX H chain, 1 copy for the FX H chain, and 2 copies for the common L chain (Fig. 1). The obtained plasmids were introduced into host cells derived from CHO cells DXB11 by electroporation. Electroporation was performed using Nucleofector(R). The cells were then cultured in the presence of 15 nmol/L of MTX to select the expression plasmid-introduced cells.

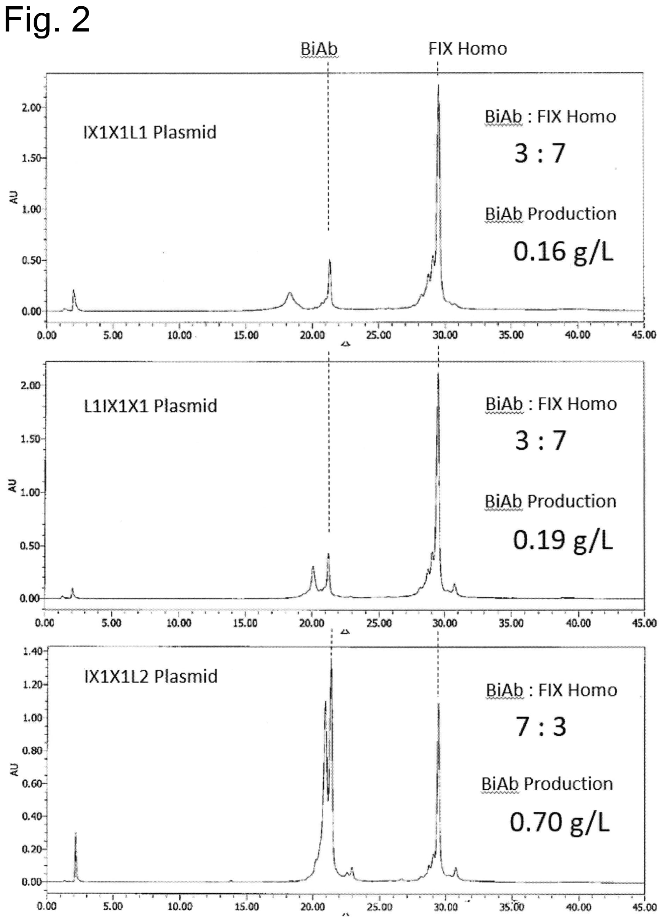

A IX1X1L1 plasmid-introduced cell pool, a L1IX1X1 plasmid-introduced cell pool, and a IX1X1L2 plasmid-introduced cell pool were obtained, and they were compared using fed-batch culture in a 24-well plate. The culture was performed under the conditions of a medium volume of 0.8 mL, a culture temperature of 37°C, and a shaking speed of 160 rpm. On the 14th day after the start of culture, the concentration of antibodies in the culture medium was measured, and the production ratio of the desired heteromeric form was evaluated by IEC. As a result, it was found that the antibody production amount and the production ratio of the desired heteromeric form in the IX1X1L2 plasmid-introduced cell pool were higher than those in the IX1X1L1 plasmid-introduced cell pool or L1IX1X1 plasmid-introduced cell pool (Fig. 2). This result suggests that the common L chain gene is underexpressed with 1 copy, and by increasing its copy number, the expression balance of each antibody chain is adjusted due to the gene dosage effect, which may have favorably influenced the amount of antibody production and the production ratio of the desired heteromeric form.

### Example 2: Gene introduction using secondary plasmid (X1 L2)

In Fig. 2 of Example 1, the peak of the FX H chain homomeric form was hardly observed, suggesting a potential deficiency in the expression level of the FX H chain. Additionally, a potential room for increased expression level of the common L chain was suggested. Then, an X1L2 plasmid consisting of 1 copy of FX H chain and 2 copies of common L chain was constructed and additionally introduced into the IX1X1L2 plasmid-introduced cell, and compared with cells into which only the IX1X1L2 plasmid was introduced.

First, the IX1X1L2 plasmid was introduced into the CHO cell DXB11-derived host cells by electroporation, and cultured in the presence of 15 nmol/L MTX to obtain a cell pool exhibiting 15 nmol/L MTX resistance. Into this cell pool, the newly constructed X1L2 plasmid was additionally introduced via electroporation, then cultured once under the presence of 15 nmol/L MTX and 200 µg/mL Hygromycin, and further cultured under the presence of 100 nmol/L MTX to obtain a cell pool exhibiting 100 nmol/L MTX resistance.

Among the resulting cell pools, two types of cell pools (A01 and A07) into which only the IX1X1L2 plasmid were introduced and cell pools obtained by additionally introducing the X1L2 plasmid into the cell pools A01 and A07 were compared using fed-batch culture. Fed-batch culture was performed using a 96-well plate with a culture medium volume of 80 µL, a culture temperature of 37°C, and a shaking speed of 250 rpm. As a result of evaluating the production ratio of the desired heteromeric form in the culture medium on the 14th day of culturing using IEC, it was found that the heteromeric form production ratio in the cell pool obtained by introducing the X1L2 plasmid in addition to the IX1X1L2 plasmid was higher than that of the cell pool into which only the IX1X1L2 plasmid was introduced. Specifically, when the X1L2 plasmid was additionally introduced into the cell pool A01, 15 out of the 17 cell pools obtained through the additional introduction had a higher production ratio of the desired heteromeric form than A01. Similarly, when the X1L2 plasmid was additionally introduced into the cell pool A07, all 10 cell pools obtained through the additional introduction had a higher production ratio of the desired heteromeric form than A07. These results suggest that the expression balance of each antibody gene can be improved by additionally introducing each gene for the FX H chain and the common L chain.

All publications, patents, and patent applications cited herein are incorporated herein by reference in their entirety.

## Claims

1. A method for generating a cell that produces a multispecific binding molecule, the method comprising a step of introducing a first vector and a second vector into a cell, wherein the first vector contains genes for all polypeptide chains constituting the multispecific binding molecule, the second vector contains a gene for a polypeptide chain with low expression, and the multispecific binding molecule may be a multispecific antibody.

2. The method according to claim 1, wherein the multispecific binding molecule is a bispecific antibody, and the bispecific antibody is an IgG-type antibody with an H chain or L chain made common.

3. A method for generating a cell that produces a bispecific antibody with a common L chain, the method comprising a step of introducing a first vector and a second vector into a cell, wherein the first vector contains genes for all polypeptide chains constituting the bispecific antibody, and the second vector contains a gene for a polypeptide chain with low expression of the bispecific antibody.

4. The method according to claim 3, wherein the polypeptide chain with low expression is one of two different H chains, and the second vector contains a nucleic acid sequence encoding the H chain, and the second vector further contains a nucleic acid sequence encoding the common L chain.

5. The method according to claim 4, wherein the second vector contains a greater number of the nucleic acid sequence encoding the common L chain than the nucleic acid sequence encoding the H chain.

6. The method according to claim 5, wherein the first vector contains one copy of a nucleic acid sequence encoding a first H chain, one copy of a nucleic acid sequence encoding a second H chain, and one or two copies of the nucleic acid sequence encoding the common L chain, and the second vector contains one copy of a nucleic acid sequence encoding an H chain with lower expression and two or more copies of the nucleic acid sequence encoding the common L chain.

7. A cell that produces an antigen-binding molecule, the cell comprising a first vector and a second vector, wherein the first vector contains genes for all polypeptide chains constituting the antigen-binding molecule, the second vector contains a gene for a polypeptide chain with low expression, the antigen-binding molecule may be multispecific, and the multispecific antigen-binding molecule may be a bispecific antibody with an H chain or L chain made common.

8. A cell that produces a bispecific antibody with a common L chain, the cell comprising a first vector and a second vector, wherein the first vector contains genes for all polypeptide chains constituting the bispecific antibody, and the second vector contains a gene for an H chain with lower expression and a gene for the common L chain.

9. The cell according to claim 8, wherein the second vector contains the gene for the common L chain at a higher copy number than the gene for the H chain.

10. The cell according to claim 9, wherein the first vector contains one copy of a gene for a first H chain, one copy of a gene for a second H chain, and one or two copies of the gene for the common L chain, and the second vector contains one copy of the gene for an H chain with lower expression and two or more copies of the gene for the common L chain.

11. A method for producing an antigen-binding molecule, the method comprising producing the antigen-binding molecule using the cell according to claim 7, or a method for producing a bispecific antibody, the method comprising producing the bispecific antibody using the cell according to claim 8.

12. The method for generating a cell according to any one of claims 1 to 6 or the production method according to claim 11, wherein the cell is a mammalian cell.

13. The method for generating a cell according to any one of claims 3 to 6 or the production method according to claim 11, wherein the bispecific antibody is a bispecific antibody against FIX(a)/FX.

14. The method according to claim 13, wherein the bispecific antibody is emicizumab.

15. A method for producing a medicament containing an antigen-binding molecule or a bispecific antibody produced by the method according to claim 11.
